(19) Europäisches Patentamt
European Patent Office
Office européen des brevets

(11) Publication number: **0 133 248 B1**

(12) **EUROPEAN PATENT SPECIFICATION**

(45) Date of publication of patent specification: **21.11.91**

(51) Int. Cl.5: **C07D 405/06**, C07D 411/06, A61K 31/41, A61K 31/335, A61K 31/39

(21) Application number: **84108548.3**

(22) Date of filing: **19.07.84**

(54) **1,2,4-Triazol-1-yl-derivatives.**

(30) Priority: **20.07.83 JP 133040/83**

(43) Date of publication of application: **20.02.85 Bulletin 85/08**

(45) Publication of the grant of the patent: **21.11.91 Bulletin 91/47**

(84) Designated Contracting States: **BE CH DE FR IT LI NL SE**

(56) References cited:
**EP-A- 0 094 167**
**EP-A- 0 106 515**
**EP-A- 0 112 284**
**GB-A- 2 095 236**

**PATENT ABSTRACTS OF JAPAN, vol. 9, no. 78 (C-274)[1801], 6th April 1985; & JP - A - 59 212 491 (SUMITOMO KAGAKU KOGYO) 01-12-1984**

(73) Proprietor: **SHIONOGI & CO., LTD.**
**1-8, Doshomachi 3-chome Chuo-ku**
**Osaka 541(JP)**

(72) Inventor: **Ogata, Masaru**
**8-20.8, Sumiyoshi-Yamate**
**Higashinada-ku Kobe-shi Hyogo(JP)**
Inventor: **Matsumoto, Hiroshi**
**5-10-25, Yamatedai**
**Ibaraki-shi Osaka(JP)**
Inventor: **Kida, Shiro**
**1-17 1-607, Daido**
**Higashiyodogawa-ku Osaka-shi Osaka(JP)**
Inventor: **Tawara, Katsuya**
**1-1-814, Higashiota**
**Ibaraki-shi Osaka(JP)**

(74) Representative: **Bruin, Cornelis Willem et al**
**OCTROOIBUREAU ARNOLD & SIEDSMA 1, Sweelinckplein**
**NL-2517 GK Den Haag(NL)**

EP 0 133 248 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

The present invention relates to 1,2,4-triazol-1-yl derivatives.

Some derivatives of this type have been disclosed earlier. Thus, GB-A-2095236 discloses derivatives of the formula

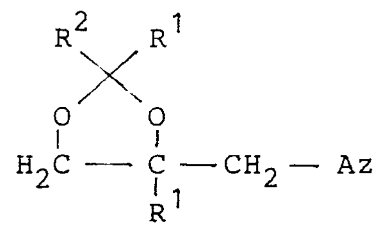

wherein Az is an 1,2,4-triazol-1-yl, 1,3,4-triazol-1-yl, or imidazol-1-yl group, $R^1$ is hydrogen, alkyl, cycloalkyl, aryl or aralkyl, $R^2$ and $R^3$ may be the same of different and each may be hydrogen, alkyl or aryl, or $R^2$ and $R^3$ together are a polymethylene group. These derivatives as well as their salts and metal complexes are reported to be active fungicides and plant growth regulators and will normally be used for agricultural and horticultural purposes. A utilisation for the treatment of candidiasis and human dermatophyte infections is mentioned in passing in said document.

Document EP-A-0094167 published on 16 November 1983 discloses derivatives of the formula

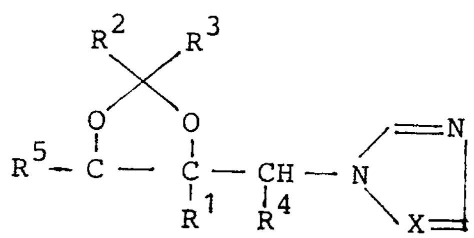

wherein X is CH or N, $R^1$ is phenyl or substituted phenyl, each of $R^2$ and $R^3$ is hydrogen or alkyl or phenyl or $R^2$ and $R^3$ together are a $C_4$-$C_8$ alkylene group, and each of $R^4$ and $R^5$ is hydrogen or alkyl. These derivatives are used as fungicides or plant growth regulators.

The present invention relates to 1,2,4-triazol-1-yl derivatives which are useful as antimycotic agents. These derivatives are represented by the formula (I)

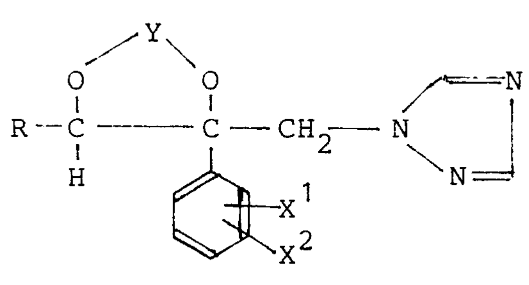

(I)

wherein

| | |
|---|---|
| R | is $C_1$-$C_5$ alkyl or phenyl optionally having 1 to 3 substituents, selected from halogen, $C_1$-$C_5$ alkyl, $C_1$-$C_5$ alkoxy; |
| $X^1$ and $X^2$ | each are hydrogen, halogen, $C_1$-$C_5$ alkyl, $C_1$-$C_5$ alkoxy; and |
| Y | is C=O, C=S or S=O; |

and include acid-addition salts thereof. They were found to have a potent antimycotic activity when administered orally.

It should be noted that azole type dioxolane derivatives having an antimycotic activity in addition to anxiolytic and/or anticonvulsive activities are disclosed in EP-A-0112284 (published 27 June 1984). These derivatives have a specific configuration of the dioxolane ring, however. Further, triazole derivatives useful as human and agricultural fungicides are disclosed in EP-A-0106515 (published on 25 April 1984). In these derivatives, a dioxolane ring is missing. Moreover, ketoconazole (US-A-4144346 and US-A-4223036) has already been used as an oral antimycotic agent but some adverse reactions thereof such as hepatic dysfunction have been reported.

The objective compounds of formula (I) can be prepared according to the following reaction sequence:

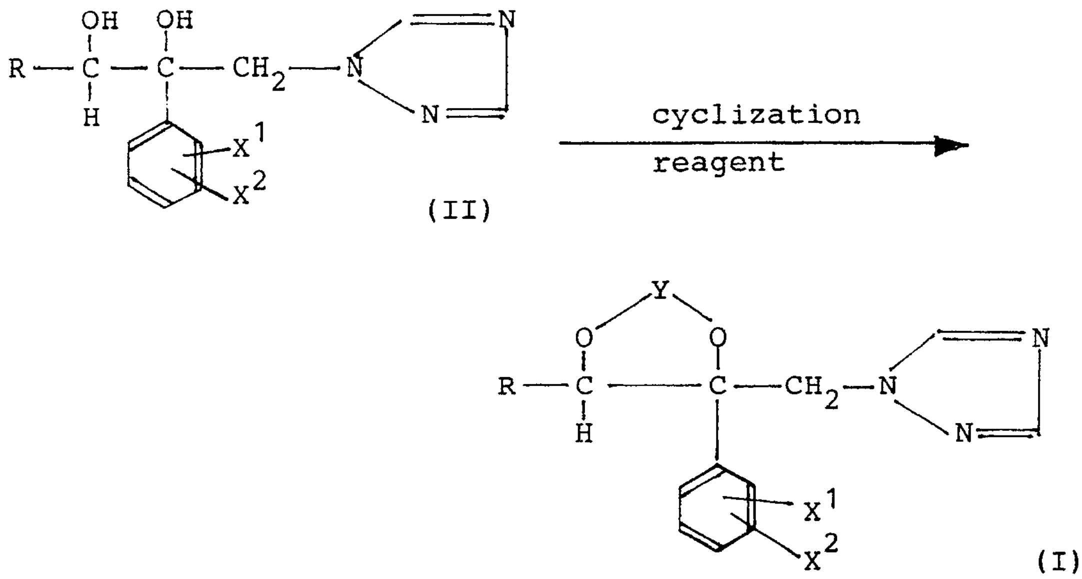


(II)

(I)

wherein R, $X^1$, $X^2$ and Y have the same meanings as defined above.

The objective compounds (I) can be produced by reacting the starting materials (II) with a cyclization reagent which is 1,1'-carbonyldiimidazole or 1,1'-thiocarbonyldiimidazole, a thionyl halide such as thionyl chloride or thionyl bromide, phosgene or thiophosgene, oxalyl chloride and a halocarbonic acid ester such as ethyl chlorocarbonate or phenyl chlorocarbonate. If necessary, an acid such as p-toluenesulfonic acid or hydrochloric acid may be used for accelerating the reaction. The reaction may be carried out in an appropriate solvent (e.g. chloroform, carbon tetrachloride, 1,2-dichloroethane, methylene chloride, dimethylformamide, benzene, or toluene) if necessary, in the presence of a base (e.g., sodium hydride, triethylamine, pyridine, 2,6-lutidine, or $\gamma$-picoline) at a temperature of 15° C to 130° C, preferably at a temperature from room temperature to the boiling point of the solvent.

Since the compounds (I) contain two or three asymmetrical carbon atoms, they are generally prepared as a mixture of diastereomers which can be separated into the respective diastereomers in a conventional manner.

The starting materials (II) can be prepared, for example, according to the following reaction sequence,

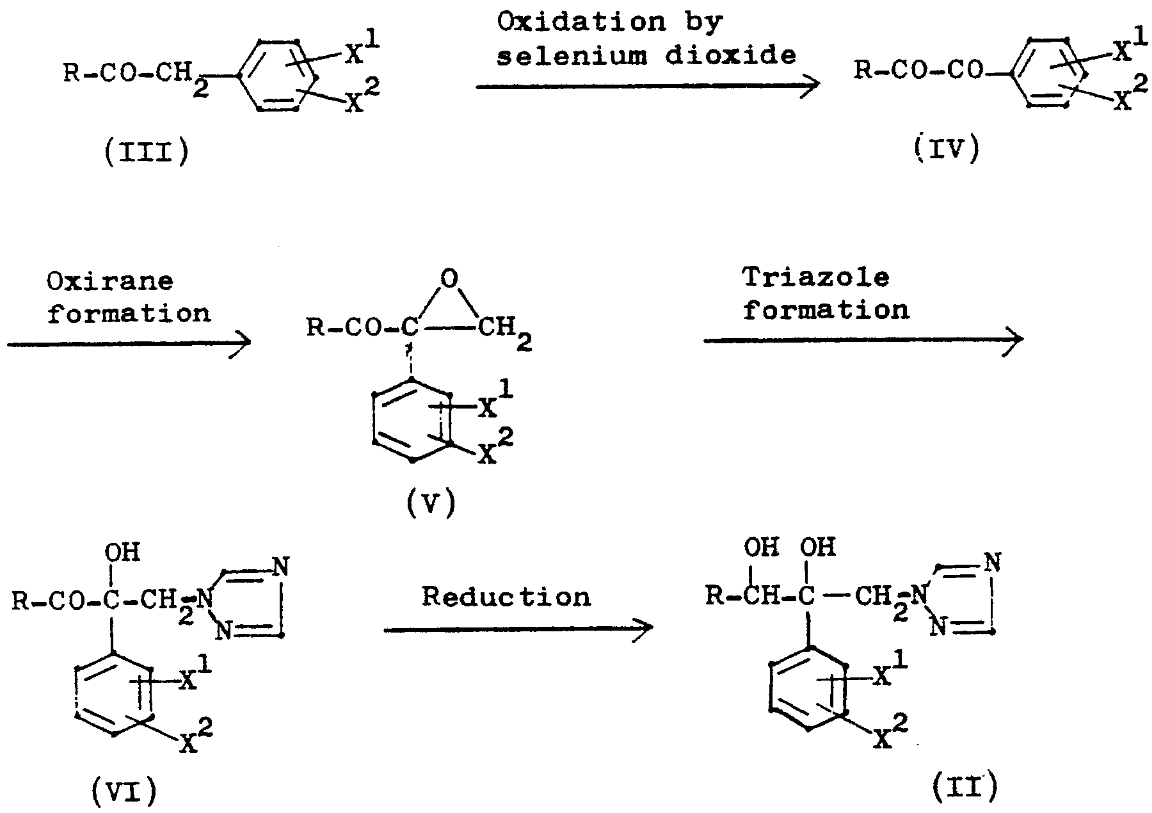


(UK. Pat Appln. No. 8,404,426)

wherein R, $X^1$, $X^2$, and Y have the same meanings as defined above.

Preferred compounds among the objective compounds (I) are those of formula (I) wherein R is $C_3$-$C_4$ alkyl or phenyl substituted by 1 or 2 halogen atoms; at least one of $X^1$ and $X^2$ is halogen; and Y is C=O, C=S or S=O.

The objective compounds (I) obtained by the above reaction sequence can be converted to pharmaceutically acceptable acid addition salts thereof. Examples of the acids which can form these salts are organic acids such as acetic acid, citric acid, tartaric acid, malic acid, succinic acid, malic acid, fumaric acid, and methanesulfonic acid and inorganic acids such as hydrohalogenic acids, sulfuric acid, and phosphoric acid.

The objective compounds (I) and their salts show potent antimycotic activity and are useful as antimycotic agents for medical or veterinary use. Particularly, the objective compounds (I) are useful as oral or injectable antimycotic agents.

The objective compounds (I) and their pharmaceutically acceptable acid-addition salts can be used alone or together with additives such as carriers, excipients, diluents, dispersants and the like in dosage forms for internal or external use. These dosage forms includes solutions, suspensions, powders, pills, granules, capsules, tablets, injections, ointments, tinctures, suppositories, and the like; and can be prepared in a conventional manner for formulation. The compounds (I) can be administered orally to a human adult at a dose or doses of 10 - 2,000 mg per day.

Furthermore, the objective compounds (I) and their acid addition salts can be expected to be fungicides when used in agricultural crops such as fruit trees, rice, wheat, cotton, corn or soya bean.

The present invention will be illustrated in detail by the following Examples.

Example 1

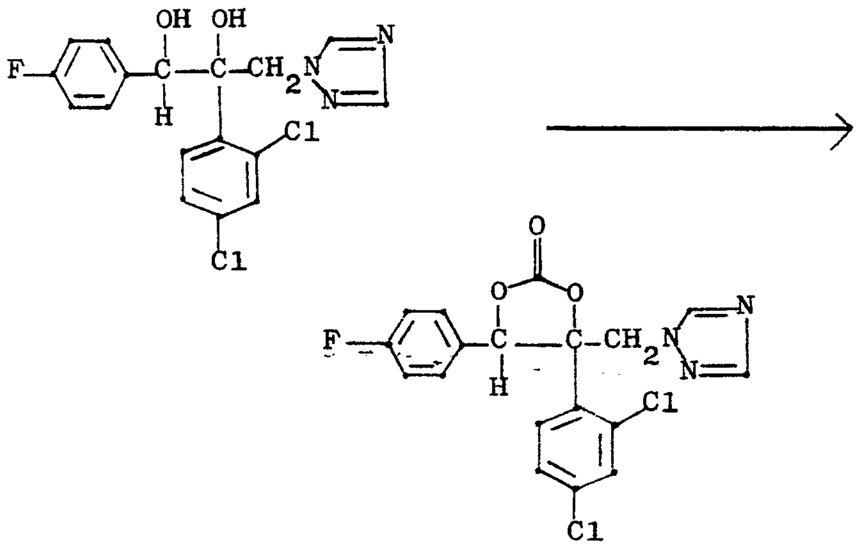

A mixture of 500 mg of 2-(2,4-dichlorophenyl)-1-(4-fluorophenyl)-3-(1H-1,2,4-triazol-1-yl)-1,2-propanediol, 530 mg of 1,1'-carbonyldiimidazole and 10 ml of dry chloroform is heated under reflux for 1 hour. Water is added to the reaction mixture, and the chloroform layer is separated, dried over anhydrous sodium sulfate, and concentrated. The residue is chromatographed on a column of silica-gel and eluted with a mixture of methylene chloride and methanol (100 : 0 - 98 : 2 v/v). The eluate is concentrated, and the residue is crystallized from ethyl acetate-isopropyl ether to give 420 mg of 4-(2,4-diclorophenyl)-5-(4-fluorophenyl)-2-oxo-4-(1H-1,2,4-triazol-1-yl)methyl-1,3-dioxolane as crystals. m.p. 191 - 192°C. Yield: 78.7 %.
Anal. Calcd. (%) (for $C_{18}H_{12}N_3O_3Cl_2F$)
: C, 52.96; H, 2.96; N, 10.29; Cl, 17.37; F, 4.65
Found (%): C, 53.35; H, 3.15; N, 10.42; Cl, 17.34 F, 4.84

IR $\nu_{max}^{Nujol}$ 1810 $cm^{-1}$ (C=O)
NMR $\delta^{CDCl_3}$ 4.08 (d, J=15Hz, 1H) } $-CH_2-$
4.93 (d, J=15Hz, 1H) }
6.08 (s, 1H, -O-CH<),
7.03 - 7.90 (m, 9H, arom.H)

Example 2 - 15

The following starting materials (II) are allowed to react with 1,1'-carbonyldiimidazole in the same manner as in Example 1 to give the corresponding objective compounds (Ia).

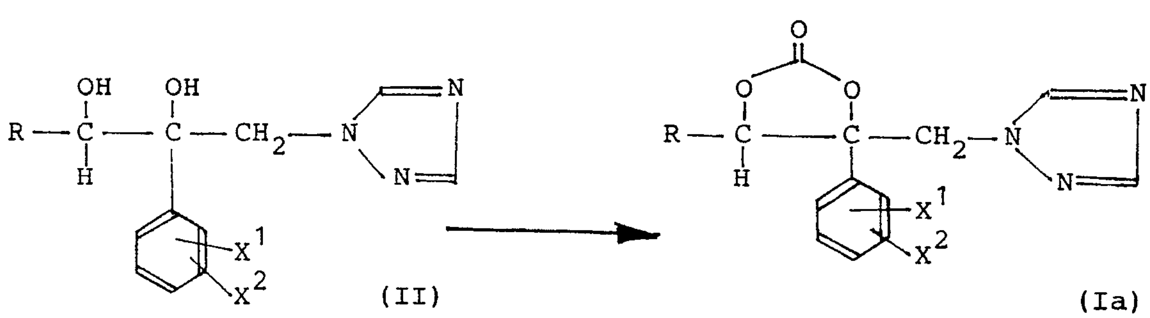

(II) (Ia)

The meanings of R, $X^1$, $X^2$ in the formulae, as well as melting points and yields of the resulting products have been indicated in the following Table.

| Ex. No. | R | $X^1$ | $X^2$ | m.p. (°C) | Yield (%) |
|---|---|---|---|---|---|
| 2 | i-Pr | 2-Cl | 4-Cl | 150-150.5 | 52.3 |
| 3 | Ph | 2-Cl | 4-Cl | 138-139 | 34.3 |
| 4 | Bu | 2-Cl | 4-Cl | 137-138 | 42.3 |
| 5 | 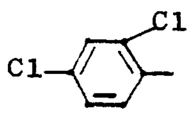 | 4-Cl | H | 162-163 | 78.2 |
| 6 | 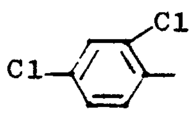 | 2-Cl | 4-Cl | 222-223 | 64.4 |
| 7 | 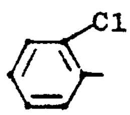 | 2-Cl | H | 234-235 | 93.4 |
| 8 | 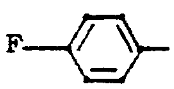 | 4-F | H | 81-82 | 69.4 |
| 9 | 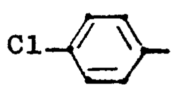 | 4-Cl | H | 155-156 | 70.1 |
| 10 | 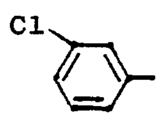 | 3-Cl | H | 148.5-149.5 | 57.9 |
| 11 | 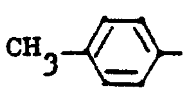 | $4-CH_3$ | H | 139-140 | 79.6 |
| 12 | Pr | 2-Cl | 4-Cl | 179-180 | 70.1 |
| 13 | Pr | 4-Cl | H | 121-122 | 69.0 |
| 14 | Ph | 2-Cl | H | 220-222 | 20.4 |
| 15 | 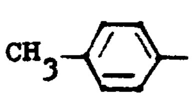 | 2-Cl | H | 195-196 | 26.0 |

Note: The abbreviations in the table have the following meanings :

Ph(Phenyl), Pr(n-propyl), i-Pr(isopropyl), Bu(n-butyl).

Example 16

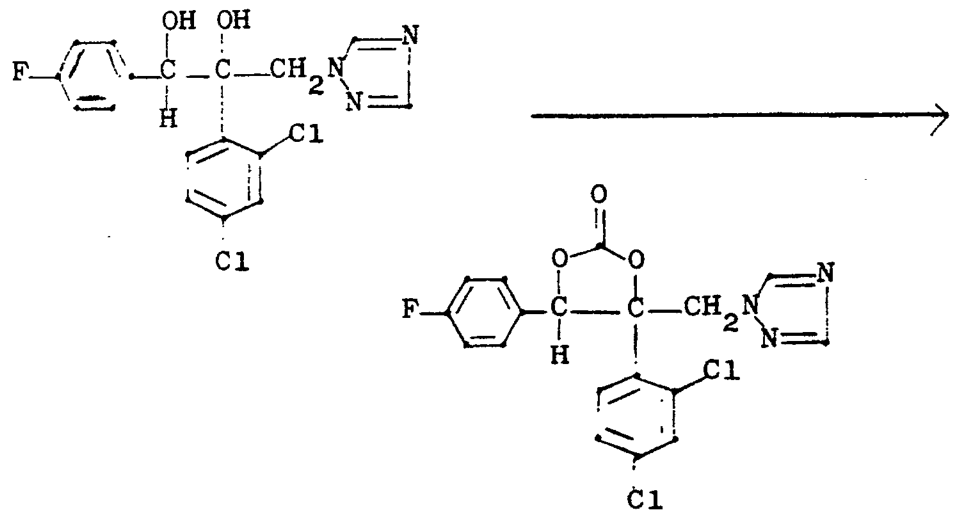

To a solution of 400 mg of 2-(2,4-dichlorophenyl)-1-(4-fluorophenyl)-3-(1H-1,2,4-triazol-1-yl)-1,2-propanediol and 430 mg of triethylamine in 20 ml of dry chloroform is added a solution of 532 mg of oxalyl chloride in 2 ml of dry chloroform in small portions with ice-cooling, and the mixture is stirred at room temperature for 1 hour. The reaction mixture is mixed with ice-water and aqueous sodium bicarbonate solution and extracted with methylene chloride. The organic layer is washed with water, dried over anhydrous sodium sulfate, and concentrated. The residue is chromatographed on a column of silica-gel and eluted with benzene-ethyl acetate (1 : 1 v/v). The eluate is concentrated, and the resulting residue is crystallized from ethyl acetate-isopropyl ether to give 219 mg of 4-(2,4-diclorophenyl)-5-(4-fluorophenyl)-2-oxo-4-(1H-1,2,4-triazol-1-yl)methyl-1,3-dioxolane as crystals. m.p. 190 - 191 °C. Yield: 51.3 %.

Example 17 - 21

The following starting materials (II) are allowed to react with oxalyl chloride in the same manner as in Example 16 to give the corresponding objective compounds (Ia).

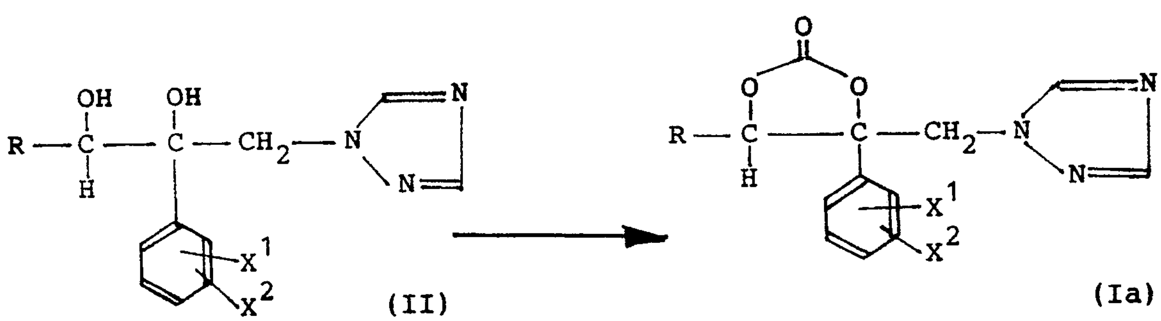
(II) (Ia)

The meanings of R, $X^1$, $X^2$ in formulae II and Ia, as well as melting points and yields of the resulting products have been represented in the following Table:

| Ex. No. | R | $X^1$ | $X^2$ | m.p. (°C) | Yield (%) |
|---|---|---|---|---|---|
| 17A | i-Pr | 4-Cl | H | 104-105 | 52 |
| B | i-Pr | 4-Cl | H | 200-201 | 82 |
| 18 | 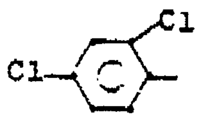 | H | H | 145-146 | 64 |
| 19 | 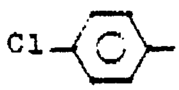 | 2-Cl | 4-Cl | 178-179 | 86 |
| 20 | 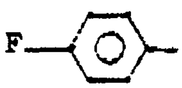 | 4-Cl | H | 176-177 | 83 |
| 21 | 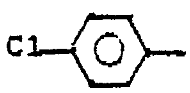 | 2-Cl | 4-Cl | IR($CHCl_3$) 1815 $cm^{-1}$ | 47 |

Notes :
1) Compounds A and B of Example 17 are diastereomers of each other
2) Yield in Example 17 A means the yield when one diastereomer (m.p. 110 - 111°C) of the starting diol is used, and Yield in Example 17 B means the yield when another diastereomer (m.p. 149 - 151°C) is used.
3) The compounds of Example 19 and Example 24 are diastereomers to each other, just like compounds A and B of Example 17.

Example 22

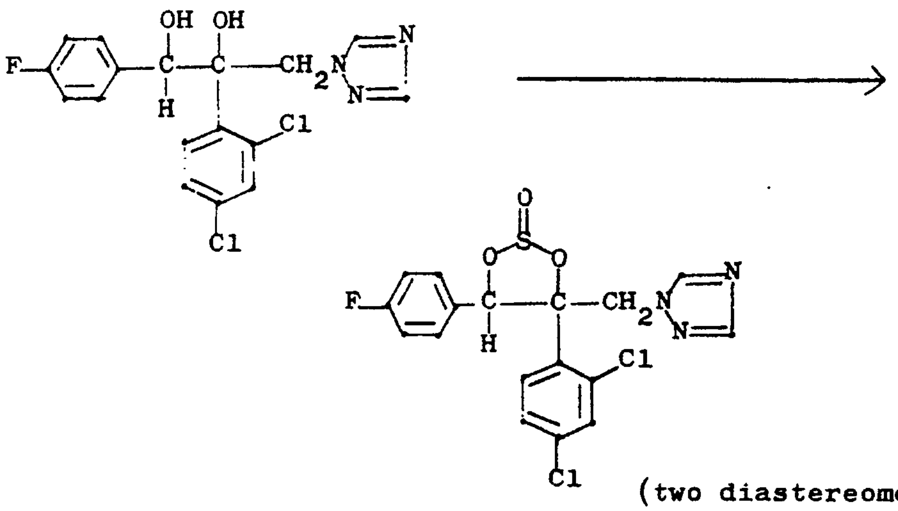

(two diastereomers)

To a solution of 400 mg of 2-(2,4-dichlorophenyl)-1-(4-fluorophenyl)-3-(1H-1,2,4-triazol-1-yl)-1,2-propanediol and 127 mg of triethylamine in 20 ml of dry chloroform is added a solution of 150 mg of thionyl chloride and 1.5 ml of dry chloroform in small portions under ice-cooling, whereupon the mixture is stirred at room temperature for 30 minutes. The reaction mixture is mixed with ice-water and aqueous sodium bicarbonate solution and is extracted with methylene chloride. The organic layer is washed with water, dried over anhydrous sodium sulfate, and concentrated. The residue is chromatographed on a column of silica-gel and eluted with benzene-ethyl acetate (1 : 1 v/v). The preliminary eluate is concentrated, and the residue is crystallized from ethyl acetate-isopropyl ether to give 158 mg of 4-(2,4-dichlorophenyl)-5-(4-fluorophenyl)-4-(1H-1,2,4-triazol-1-yl)methyl-1,3,2-dioxathiolane-2-oxide as crystals. m.p. 170 - 171 °C. Yield: 35.3 %.
Anal. Calcd. (%): (for $C_{17}H_{12}N_3O_3Cl_2SF$)
C, 47.68; H, 2.82; N, 9.81; Cl, 16.55; S, 7.49; F, 4.44
Found (%): C, 47.55; H, 2.96; N, 9.84; Cl, 16.51; S, 7.61; F. 4.73

IR $\nu_{max}^{Nujol}$ : 1230, 1170 $cm^{-1}$
NMR $\delta^{d_6\text{-DMSO}}$: 4.30(d,J=16.5Hz,1H) } $-CH_2-$
4.98(d,J=16.5Hz,1H) }
6.45(s,1H,-O-CH<),
7.65(s,1H) } triazole H
8.27(s,1H) }
7.25-8.05(m,7H,arom.H)

The subsequent eluate is concentrated, and the residue is crystallized from methanol to give 134 mg of the diastereomer of the above product as crystals. m.p. 237 - 239 °C. Yield: 29.9 %.
Anal. Calcd. (%): (for $C_{17}H_{12}N_3O_3Cl_2SF$)
C, 47.68; H, 2.82; N, 9.81; Cl, 16.55; S, 7.49; F, 4.44
Found (%): C, 47.53; H, 2.96; N, 9.71; Cl, 16.45; S, 7.61; F, 4.67

IR $\nu_{max}^{Nujol}$ : 1215, 985 $cm^{-1}$
NMR $\delta^{d6\text{-DMSO}}$ : 4.35(d,J=16.5Hz,1H) $-CH_2-$
4.82(d,J=16.5Hz,1H)
6.75(s,1H,-O-CH )
8.32(s,1H,triazole H)
7.25-7.70(m,8H,arom H and triazole H)

Example 23 - 26

The following starting materials (II) are allowed to react with thionyl chloride in the same manner as in Example 25 to give the corresponding objective compounds (Ib).

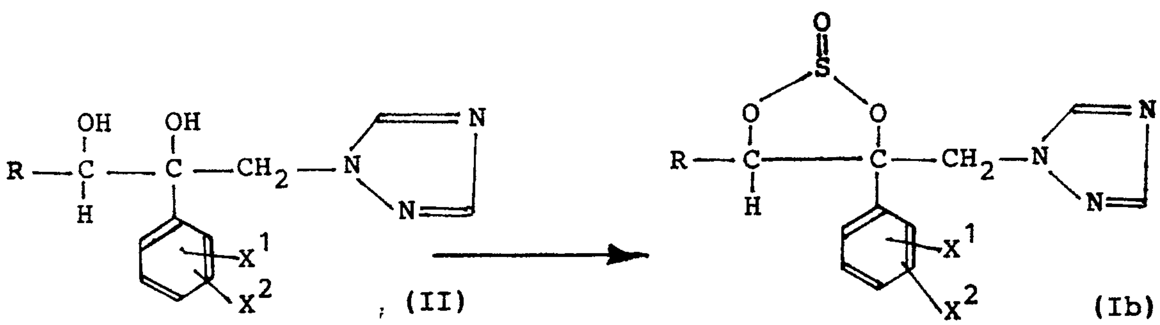

; (II) (Ib)

**two diastereomers**

| Ex. No. | R | $X^1$ | $X^2$ | m.p. (°C) | Yield (%) |
|---|---|---|---|---|---|
| 23A | i-Pr | 4-Cl | H | 157-158 | 23 |
| B | i-Pr | 4-Cl | H | 120-121 | 20 |
| 24A | 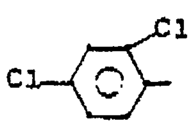 | 4-Cl | H | 163.5-164.5 | 19 |
| B | 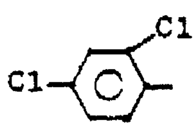 | 4-Cl | H | 108-109 | 28 |
| 25A | Ph | 2-Cl | 4-Cl | 179-180 | 27 |
| B | Ph | 2-Cl | 4-Cl | 231-233 | 16 |
| 26A | 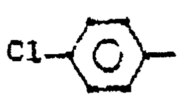 | 2-Cl | 4-Cl | 187-188 | 4 |
| B | 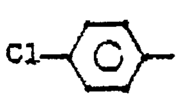 | 2-Cl | 4-Cl | 235-236 | 35 |

Example 27

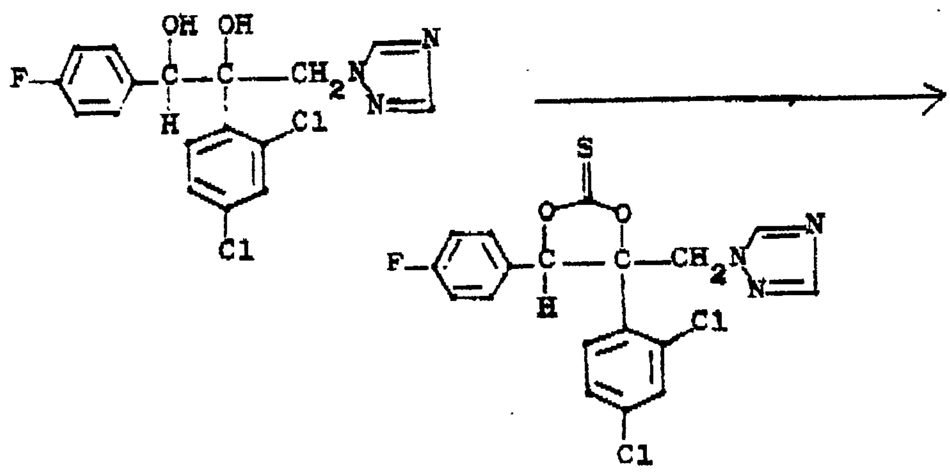

A solution of 500 mg of 2-(2,4-dichlorophenyl)-1-(4-fluorophenyl)-3-(1H-1,2,4-triazol-1-yl)-1,2-propanediol and, 700 mg of 1,1'-thiocarbonyldiimidazole in 10 ml of dry chloroform is heated under reflux for 1 hour. To the reaction mixture is added ice-water, and the mixture is extracted with chloroform. The chloroform layer is washed with water, dried over anhydrous sodium sulfate, and concentrated. The residue is chromatographed on a column of silica-gel and eluted with benzene-ethyl acetate (4 : 1 v/v). The eluates containing the objective compound are combined and concentrated. The residue is washed with isopropyl ether to give crystalline materials. The crystals are recrystallized from ethyl acetate-isopropyl ether to give 360 mg of 4-(2,4-dichlorophenyl)-5-(4-fluorophenyl)-2-thioxo-4-(1H-1,2,4-triazol-1-yl)methyl-1,3-dioxolane. m.p. 169 - 170° C. Yield: 64.9 %.

$$\mathrm{IR}\ \nu_{max}^{Nujol}\ :\ 1303,\ 1290\ cm^{-1}\ (C{=}S)$$

Anal. Calcd. (%) (for $C_{18}H_{12}N_3O_2Cl_2FS$)
C, 50.96; H, 2.85; N, 9.90; Cl, 16.71; F, 4.48; S, 7.56
Found (%) : C, 50.91, H, 2.98; N, 9.97; Cl, 16.51; F, 4.77; S, 7.69

NMR $\delta^{CDCl_3}$ 4.20(d, J=16.5Hz, 1H)} $-CH_2-$
4.97(d, J=16.5Hz, 1H)}
6.28(s, 1H), -O-CH-
7.59(s, 1H)} triazole H
7.98(s, 1H)}
7.15-7.60(m, 7H, arom.H)

Example 28

To a solution of 500 mg of 1-(4-chlorophenyl)-2-(2,4-dichlorophenyl)-3-(1H-1,2,4-triazol-1-yl)-1,2-propanediol in 1 ml of dry pyridine is added 300 mg of phenyl chlorocarbonate, and the resultant mixture is allowed to react at room temperature for 18 hours. The reaction mixture is mixed with aqueous sodium hydrogencarbonate solution and shaken with methylene chloride. The organic layer is washed with water, dried over anhydrous sodium sulfate and concentrated. The residue is chromatographed on a column of silica-gel, which is eluted with 2 % methanolmethylene chloride. The eluate is concentrated and the residue is crystallized from ethyl acetate-isopropyl ether to give 466 mg of 5-(4-chlorophenyl)-4-(2,4-dichlorophenyl)-2-oxo-4-(1H-1,2,4-triazol-1-yl)methyl-1,3-dioxolane as crystals melting at 178 to 179° C. The yield is 87 %.

Example 29

The reaction of Example 28 is repeated except that the phenyl chloroformate is replaced by ethyl chlorocarbonate. The resultant product is crystallized from ethyl acetate isopropyl ether to give the same product. The yield is 50 %.

The compounds used in the following Experiments are represented by the numbers which correspond to the numbers of the respective Examples.

Experiment 1

Antimycotic test against Trichophyton asteroides

The Minimum Inhibitory Concentration (MIC, μg/ml) of each test compound against Trichophyton asteroides in an in vitro antimycotic test is shown below. Sabouraud's dextrose broth *was used as a culture medium.

| Compound Nos. | MIC (μg/ml) |
|---|---|
| 1 | 0.1 |
| 2 | 3.1 |
| 3 | 0.1 |
| 4 | 0.2 |
| 8 | 3.1 |
| 12 | 1.6 |
| 15 | 3.1 |
| 19 | 0.2 |
| 24B | 0.1 |

Experiment 2

Test on inhibition of pseudohyphae formation in Candida albicans

To Eagle MEN broth (Nissan 2, Nissui Seiyaku Co., Ltd.) was added 20 % bovine serum whereupon the broth was inoculated with Candida albicans KE-2 yeast cells at a rate of 1 x $10^6$ cells/ml (final inoculum size), and each test compound was added thereto by means of a two-fold serial dilution method. After being cultivated at 37 °C for 18 hours, the organisms of each serial dilution were smeared and fixed on a slide glass and stained by Giemsa's staining, whereupon the occurrence of pseudohyphae was observed under a microscope. The minimum concentration which could inhibit the formation of pseudohyphae was regarded as the inhibitory concentration of the compound against pseudohyphae formation.

| Compound No. | Inhibitory concentration against pseudohyphae formation (μg/ml) |
|---|---|
| 1 | 0.31 |
| 2 | 0.63 |
| 5 | 0.63 |
| 7 | 0.31 |
| 11 | 1.25 |
| 15 | 0.63 |
| 19 | 0.04 |
| 24B | 0.02 |

Experiment 3

Therapeutic effect against experimental candidasis in mice

* Totani et al., J. Med. Chem., 24, (12) 1492 (1981)

Candida albicans KE-2 was cultured in Sabouraud's dextrose agar at 28°C for 48 hours, and the resulting culture was suspended in Sabouraud's dextrose broth. The resulting cells (5 x $10^5$) were intravenously administered to the tail of Jcl-ICR female mice (4 weeks age, body weight: 18 - 20 g). Each test compound suspended in 2 % gum-arabic was orally administered at a dose of 25 mg/kg twice a day for 5 days; on the first day, the test compound was administered twice, i.e., immediately after the infection and 2 hours later; on the second and later days, the test compound was administered 24 hours after the administration of the previous day, respectively. No test compound was administered to a control group after infection thereof. Eight mice were employed in the control group and in each test group to which the test compound was administered. The therapeutic effect was evaluated on the basis of the survival rate at the 15th day from the day of infection; the results are shown below.

| Compound No. | Survival rate (%) |
|---|---|
| 1 | 87.5 |
| 2 | 100 |
| 5 | 100 |
| 19 | 86 |
| 24B | 75 |
| Control | 0 |

## Claims

**1.** A compound of the formula (I)

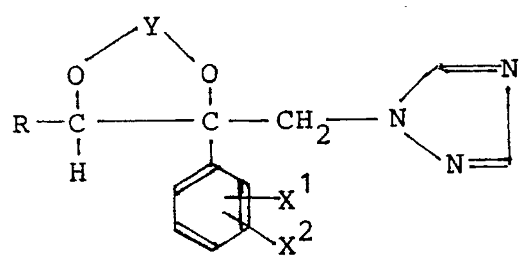

(I)

wherein
R is $C_1$-$C_5$ alkyl or phenyl optionally having 1 to 3 substituents selected from halogen, $C_1$-$C_5$ alkyl,
and $C_1$-$C_5$ alkoxy;
$X^1$ and $X^2$ each are hydrogen, halogen, $C_1$-$C_5$ alkyl, $C_1$-$C_5$ alkoxy; and
Y is C=O, C=S or S=O;
or an acid-addition salt thereof.

**2.** A compound as claimed in claim 1, in which R is $C_3$-$C_4$ alkyl or phenyl substituted by 1 or 2 halogen atoms; at least one of $X^1$ and $X^2$ is halogen.

**3.** A compound as claimed in claim 1, namely 4-(2,4-dichlorophenyl)-5-(4-fluorophenyl)-2-oxo-4-(1H-1,2,4-triazol-1-yl)methyl-1,3-dioxolane.

4. A compound as claimed in claim 1, namely 4-(2,4-dichlorophenyl)-5-isopropyl-2-oxo-4-(1H-1,2,4-triazol-1-yl)-methyl-1,3-dioxolane.

5. A compound as claimed in claim 1, namely 4-(4-chlorophenyl)-5-(2,4-dichlorophenyl)-2-oxo-4-(1H-1,2,4-triazol-1-yl)methyl-1,3-dioxolane.

6. A compound as claimed in claim 1, namely 4-(2,4-dichlorophenyl)-5-(4-fluorophenyl)-4-(1H-1,2,4-triazol-1-yl)methyl-1,3,2-dioxathiolane-2-oxide.

7. A compound as claimed in claim 1, namely 4-(2,4-dichlorophenyl)-5-(4-fluorophenyl)-2-thioxo-4-(1H-1,2,4-triazol-1-yl)methyl-1,3-dioxolane.

8. A process for preparing a compound according to claim 1 which comprises reacting a compound of the formula (II)

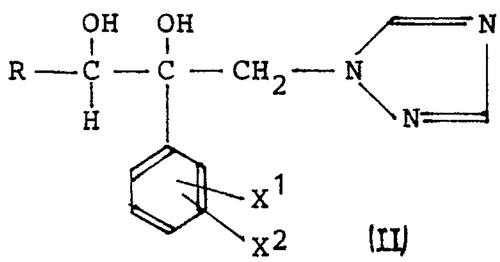

(II)

(wherein R, $X^1$ and $X^2$ have the same meanings as defined in claim 1) with a cyclization reagent selected from the group consisting of a halocarbonic acid ester, a thionyl halide, 1,1'-carbonyldiimidazole, oxalyl chloride, phosgene, 1,1'-thiocarbonyldiimidazole, and thiophosgene, if necessary in the presence of a base in an appropriate solvent at a temperature of 15° to 130° C.

9. Use of the compounds as claimed in claim 1 for preparing an antimycotic pharmaceutical composition.

**Revendications**

1. Composé de la formule (I) :

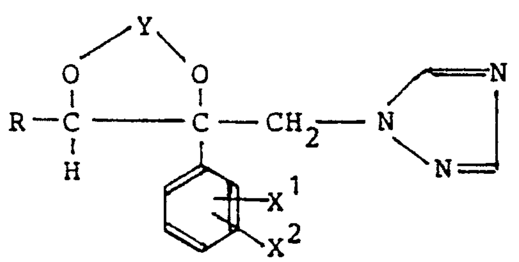

(I)

dans laquelle :

- R représente alkyle en $C_1$-$C_5$ ou phényle ayant facultativement 1 à 3 substituants choisis parmi halogène, alkyle en $C_1$-$C_5$ et alcoxy en $C_1$-$C_5$ ;
- $X^1$ et $X^2$ représentent chacun hydrogène, halogène, alkyle en $C_1$-$C_5$, alcoxy en $C_1$-$C_5$ ; et
- Y représente C=O, C=S ou S=O ; ou sel d'addition avec un acide de ce composé.

2. Composé selon la revendication 1, dans lequel R représente alkyle en $C_3$-$C_4$ ou phényle substitué par 1 ou 2 atomes d'halogène ; au moins l'un parmi $X^1$ et $X^2$ représente halogène.

3. Composé selon la revendication 1, qui est le (dichloro-2,4 phényl)-4 (fluoro-4 phényl)-5 oxo-2 (1H-triazol-1,2,4 yl-1)méthyl-4 dioxolanne-1,3.

4. Composé selon la revendication 1, qui est le (dichloro-2,4 phényl)-4 isopropyl-5 oxo-2 (1H-triazol-1,2,4 yl-1)méthyl-4 dioxolanne-1,3.

5. Composé selon la revendication 1, qui est le (chloro-4 phényl)-4 (dichloro-2,4 phényl)-5 oxo-2 (1H-triazol-1,2,4 yl-1)méthyl-4 dioxolanne-1,3.

6. Composé selon la revendication 1, qui est le (dichloro-2,4 phényl)-4 (fluoro-4 phényl)-5 (1H-triazol-1,2,4 yl-1)méthyl-4 dioxathiolanne-1,3,2 oxyde-2.

7. Composé selon la revendication 1, qui est le (dichloro-2,4 phényl)-4 (fluoro-4 phényl)-5 thioxo-2 (1H-triazol-1,2,4 yl-1)méthyl-4 dioxolanne-1,3.

8. Procédé de préparation d'un composé selon la revendication 1, qui comprend la réaction d'un composé de formule (II) :

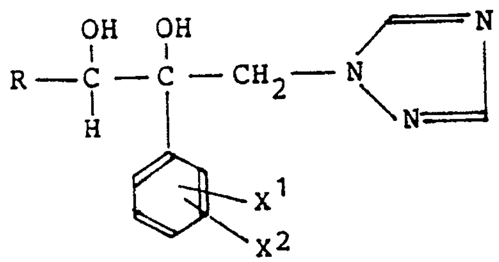

(II)

(dans laquelle R, $X^1$ et $X^2$ ont les mêmes significations que celles définies à la revendication 1) avec un réactif de cyclisation choisi dans le groupe constitué par les esters d'acides halocarboniques, les halogénures de thionyle, le carbonyldiimidazole-1,1', le chlorure d'oxalyle, le phosgène, le thiocarbonyldiimidazole-1,1', et le thiophosgène, si nécessaire en présence d'une base, dans un solvant approprié, à une température de 15° C à 130° C.

9. Utilisation des composés selon la revendication 1, pour la préparation d'une composition pharmaceutique antimycotique.

**Patentansprüche**

1. Verbindung der Formel (I):

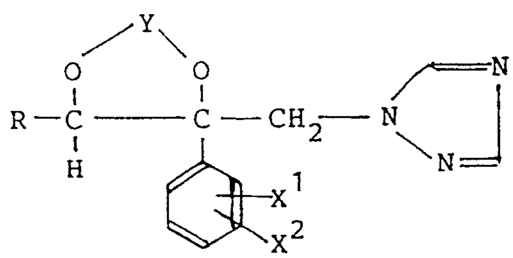

(I)

worin R $C_1$-$C_5$-Alkyl oder Phenyl, welches gegebenenfalls
1 bis 3 Substituenten, ausgewählt unter Halogen, $C_1$-$C_5$-Alkyl und $C_1$-$C_5$-Alkoxy, hat, bedeutet;
$X^1$ und $X^2$ je Wasserstoff, Halogen, $C_1$-$C_5$-Alkyl, $C_1$-$C_5$-Alkoxy bedeuten; und
Y C=O, C=S oder S=O bedeutet,
oder ein Säureadditionssalz davon.

2. Verbindung nach Anspruch 1, worin R $C_3$-$C_4$-Alkyl oder Phenyl, substituiert durch 1 oder 2 Halogenatome, bedeutet und mindestens einer der Substituenten $X^1$ und $X^2$ Halogen bedeutet.

3. Verbindung nach Anspruch 1, nämlich 4-(2,4-Dichlorphenyl)-5-(4-fluorphenyl)-2-oxo-4-(1H-1,2,4-triazol-1-yl)-methyl-1,3-dioxolan.

4. Verbindung nach Anspruch 1, nämlich 4-(2,4-Dichlorphenyl)-5-isopropyl-2-oxo-4-(1H-1,2,4-triazol-1-yl)-methyl-1,3-dioxolan.

5. Verbindung nach Anspruch 1, nämlich 4-(4-Chlorphenyl)-5-(2,4-dichlorphenyl)-2-oxo-4-(1H-1,2,4-triazol-1-yl)-methyl-1,3-dioxolan.

6. Verbindung nach Anspruch 1, nämlich 4-(2,4-Dichlorphenyl)-5-(4-fluorphenyl)-4-(1H-1,2,4-triazol-1-yl)-methyl-1,3,2-dioxathiolan-2-oxid.

7. Verbindung nach Anspruch 1, nämlich 4-(2,4-Dichlorphenyl)-5-( 4-fluorphenyl)-2-thioxo-4-(1H-1,2,4-triazol-1-yl)-methyl-1,3-dioxolan.

8. Verfahren zur Herstellung einer Verbindung nach Anspruch 1, dadurch **gekennzeichnet,** *daß* eine Verbindung der Formel (II):

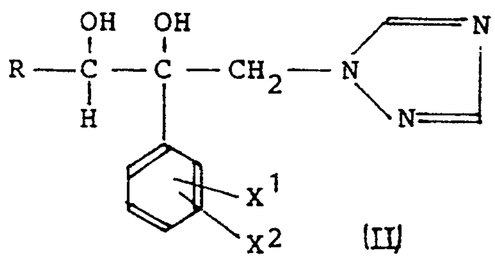

(II)

(worin R, $X^1$ und $X^2$ die in Anspruch 1 gegebenen Bedeutungen besitzen) mit einem Cyclisierungsreagens, ausgewählt aus der Gruppe bestehend aus einem Halogenkohlensäureester, einem Thionylhalogenid, 1,1'-Carbonyldiimidazol, Oxalylchlorid, Phosgen, 1,1'-Thiocarbonyldiimidazol und Thiophosgen, gegebenenfalls in Anwesenheit einer Base in einem geeigneten Lösungsmittel bei einer Temperatur von 15 bis 130° C umgesetzt wird.

9. Verwendung der Verbindungen nach Anspruch 1 für die Herstellung einer antimykotischen pharmazeutischen Zusammensetzung.